# EUROPEAN PATENT APPLICATION

(11) **EP 4 088 681 A1**
(43) Date of publication of application: **16.11.2022**
(21) Application number: 21305611.2
(22) Date of filing: 11.05.2021
(51) Int. Cl.: A61B 34/30, A61B 34/00, A61B 34/20, A61B 34/10, A61B 17/00, A61B 17/16

(54) **SYSTEM FOR GUIDING A SURGICAL TOOL COMPRISING AN OPERATIVE AXIS WITH RESPECT TO AN ANATOMICAL STRUCTURE**

(71) Applicant: DePuy Ireland Unlimited Company, County Cork (IE)
(72) Inventor: GIRARDEAU-MONTAUT, Daniel, 38610 GIERES (FR); DEMANGET, Nicolas, 38610 GIERES (FR); HUNT, Chris, LEEDS, LS11 8DT (GB)
(74) Representative: Regimbeau

(57) **Abstract**

The present disclosure relates to a system for guiding a surgical tool (1) comprising an operative axis with respect to an anatomical structure according to a planned trajectory defined by a target axis (T), comprising:
- a robotic device (100) comprising:
∘ an end effector (10) comprising the surgical tool or configured to be coupled to the surgical tool;
∘ an actuation unit (11) comprising at least three motorized degrees of freedom;
∘ a planar articulation (12) coupling the end effector (10) to the actuation unit (11), the planar articulation being configured to constrain movement of the operative axis (R) of the surgical tool inside a single plane;

- a localization device (200) configured to determine in real time a position and orientation of the operative axis (R) with respect to a coordinate system of the anatomical structure;
- a user interface (300);
- a control unit (400) coupled to the localization device (200), the actuation unit (11) and the user interface (300);
wherein the control unit (400) is configured to:
- based on the planned trajectory and on localization data from the localization device, determine a position and orientation of the operative axis (R) relative to a plane containing the target axis (T);
- control the actuation unit to constrain the operative axis (R) inside the plane containing the target axis (T) while a user moves the end effector (10) closer to the target axis (T);
- generate by the user interface (300) at least one signal related to the position and orientation of the operative axis (R) relative to the target axis (T).

## Description

### TECHNICAL FIELD

The present disclosure relates to a system for guiding a surgical tool comprising an operative axis with respect to an anatomical structure according to a planned trajectory defined by a target axis.

### TECHNICAL BACKGROUND

Some surgical interventions may be carried out with a rotary cutting tool to remove bony material from a patient's anatomical structure.

To that end, a trajectory of the rotary cutting tool intended to achieve the desired bony material removal may be planned. Said planned trajectory may be defined by a target axis, to which the rotational axis of the cutting tool has to be aligned to cut the anatomical structure.

Such a linear trajectory may be guided by a surgical robotic device having at least five motorized degrees of freedom.

However, in case the surgical robotic device comprises less than five motorized degrees of freedom, full guidance of the trajectory of the surgical tool is no longer possible.

### SUMMARY OF THE DISCLOSURE

The present disclosure relates to a system for guiding a surgical tool according to a linear trajectory with a surgical robotic device comprising at least three motorized degrees of freedom.

Some embodiments relate to a system for guiding a surgical tool comprising an operative axis with respect to an anatomical structure according to a planned trajectory defined by a target axis, comprising:
- a robotic device comprising:
   ∘ an end effector comprising the surgical tool or configured to be coupled to the surgical tool;
   ∘ an actuation unit comprising at least three motorized degrees of freedom;
   ∘ a planar articulation coupling the end effector to the actuation unit, the planar articulation being configured to constrain movement of the operative axis of the surgical tool inside a single plane;
- a localization device configured to determine in real time a position and orientation of the operative axis with respect to a coordinate system of the anatomical structure;
- a user interface;
- a control unit coupled to the localization device, the actuation unit and the user interface;
wherein the control unit is configured to:
- based on the planned trajectory and on localization data from the localization device, determine a position and orientation of the operative axis relative to a plane containing the target axis;
- control the actuation unit to constrain the operative axis inside the plane containing the target axis while a user moves the end effector closer to the target axis;
- generate by the user interface at least one signal related to the position and orientation of the operative axis relative to the target axis.

Other embodiments relate to a system for guiding a surgical tool comprising an operative axis with respect to an anatomical structure according to a planned trajectory defined by a target axis, comprising:
- a robotic device comprising:
   ∘ an end effector comprising the surgical tool configured to be coupled to the surgical tool;
   ∘ an actuation unit comprising at least three motorized degrees of freedom;
   ∘ a planar articulation coupling the end effector to the actuation unit, the planar articulation being configured to constrain movement of the operative axis of the rotary cutting tool to be perpendicular to a single plane;
- a localization device configured to determine in real time a position and orientation of the operative axis with respect to a coordinate system of the anatomical structure;
- a user interface;
- a control unit coupled to the localization device, the actuation unit and the user interface;
wherein the control unit is configured to:
- based on the planned trajectory and on localization data from the localization device, determine an orientation of the planar articulation relative to the target axis;
- control the actuation unit to constrain the plane of the planar articulation to be perpendicular to the target axis while a user moves the end effector closer to the target axis;
- generate by the user interface at least one signal related to the position of the operative axis relative to the target axis.

By "operative axis" is meant in the present text an axis of the tool defining a direction of action of the tool.

The surgical tool may be a powered tool, such as a drill, a burr or a reamer. Such a powered tool comprises a shaft coupled to a motor configured to rotate the shaft about a longitudinal axis of said shaft, and a cutting tip at a distal end of the shaft, opposite to the motor, the operative axis of the tool being the rotation axis of the shaft. Alternatively, the surgical tool may be a manual tool, such as an impactor. Such a manual tool comprises a shaft, a handle arranged at a proximal end of the shaft and a distal end opposite to the handle, the operative axis of the tool being the longitudinal axis of the shaft.

In some embodiments, the actuation unit comprises at least four motorized degrees of freedom and the control unit is configured to control the actuation unit to constrain the operative axis to be parallel to the target axis as the user moves the end effector to the target axis.

In some embodiments, the planned trajectory is further defined by a target point or area, and the control unit is configured to generate by the user interface at least one signal related to the position of the operative axis of the surgical tool relative to the target point or area.

Based on localization data from the localization device, the control unit may be configured to determine a position of a distal tip of the surgical tool relative to the target point or area.

The user interface may be configured to generate a signal related to the position of the distal tip of the surgical tool relative to the target point or area.

In some embodiments, the surgical tool is a powered tool, the target point defines an end of the trajectory of the surgical tool and the control unit is configured to switch off the surgical tool when the distal tip of the surgical tool reaches the target point.

The system may comprise a first tracker configured to be rigidly attached to the end effector or to the surgical tool and a second tracker configured to be rigidly attached to the anatomical structure.

The control unit may be configured to determine that the operative axis is aligned with the target axis.

In some embodiments, it may be considered that the operative axis is aligned with the target axis if the operative axis is at a distance of less than 1 mm from the target axis and/or the operative axis is inclined by less than 1° from the target axis.

The control unit may be configured to determine that the operative axis is aligned with the target axis when an angle between the operative axis of the cutting tool and the target axis is less than 1°.

In some embodiments, the system further comprises a lockable holding arm supporting the actuation unit.

In some embodiments, the surgical tool is a powered tool and the control unit is configured to impede switching on the rotary cutting tool as long as the operative axis is not aligned with the target axis.

In some embodiments, the surgical tool is a drill, a reamer, a burr, a screw, a K-wire, or a pin.

In some embodiments, the surgical tool is an impactor.

The end effector may advantageously comprise a guide tube adapted to alternately guide the drill, reamer or burr and the impactor.

### BRIEF DESCRIPTION OF THE DRAWINGS

FIG. 1 shows an overview of a surgical system.
FIG. 2 is a perspective view of an embodiment of the robotic device holding a rotary cutting tool with the rotation axis parallel to the plane of the planar articulation.
FIGS. 3A-3D are a schematic representation of a first embodiment of the user interface allowing guiding the rotary cutting tool in the embodiment of FIG. 2.
FIGS. 4A-4D are a schematic representation of a second embodiment of the user interface allowing guiding the rotary cutting tool in the embodiment of FIG. 2.
FIGS. 5A-5D are a schematic representation of a third embodiment of the user interface allowing guiding the rotary cutting tool in the embodiment of FIG. 2.
FIGS. 6A-6D are a schematic representation of a fourth embodiment of the user interface allowing guiding the rotary cutting tool in the embodiment of FIG. 2.
FIG. 7 is a perspective view of an embodiment of the robotic device holding a guide tube to guide the rotary cutting tool with the rotation axis perpendicular to the plane of the planar articulation.
FIGS. 8A-8C are a schematic representation of an embodiment of the user interface allowing guiding the rotary cutting tool in the embodiment of FIG. 7.
FIGS. 9A-9C are a schematic representation of a second embodiment of the user interface allowing guiding the rotary cutting tool in the embodiment of FIG. 7.
FIGS. 10A-10B are perspective views of an embodiment of the robotic device in the context of hip surgery.
FIG. 11A-11B are perspective views of an embodiment of the robotic device in the context of shoulder surgery.
FIGS. 12A-12D are schematic views of various embodiments of the planar articulation.
FIG. 13 is a flowchart describing a control loop allowing compensation of relative movements of the rotary cutting tool and the anatomical structure.

### DETAILED DESCRIPTION OF EMBODIMENTS

The present disclosure is directed to surgical interventions in which an anatomical structure, e.g. a bone, is to be treated, cut, impacted, or manipulated by a surgical tool according to a planned trajectory which is defined by a target axis.

The surgical tool has an operative axis. The operative axis may be an axis of rotation of the surgical tool, in particular if the tool is a rotary cutting tool. Alternatively, the operative axis may be an axis of a rod of the tool, defining a direction of operation of the tool, for example a direction of impaction if the tool is an impactor.

Planning of the target axis may be performed using patient's pre-operative images (e.g. CT, MRI, Ultrasound images, 2D or 3D X-rays in combination with statistical shape models, PET, etc.) or intra-operative 3D data (e.g. intra-operative CT or CBCT, intra-operative MRI, Ultrasound images, 2D or 3D intra-operative X-ray images, geometric data provided by localizing systems and providing 3D points, clouds of 3D points, surfaces reconstructed from clouds of 3D points, etc.), or both.

Multiple computer-assisted surgery methods exist to register the target axis with a coordinate system attached to the anatomical structure to be cut, using images or geometric patient data collected during surgery.

Typically, intra-operative images or data are used to register pre-operative images in a unique coordinate system attached to the anatomical structure, and usually represented by a tracker that can use any of computer assisted surgery technologies (optical tracker made of reflective markers, optical tracker made of active LEDs, electromagnetic trackers made of coils, combination of inertial sensors, ultrasonic sensors, RFID sensors, etc.).

Using any of these conventional computer-assisted surgery methods results in that the target axis has a known geometric representation in a coordinate system attached to the anatomical structure to be cut, and whose movements are tracked in real-time by a tracking unit as it will be detailed below.

### General overview of the system

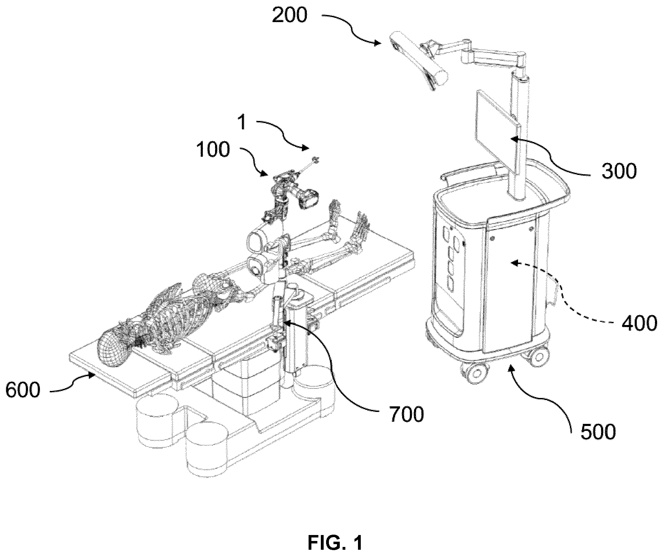

FIG. 1 shows an overview of a surgical system according to the invention.

A patient is lying on an operating table 600, e.g. in view of hip surgery.

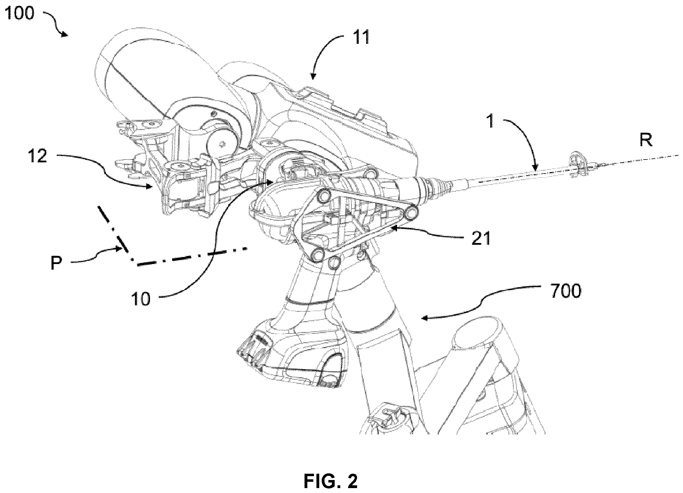

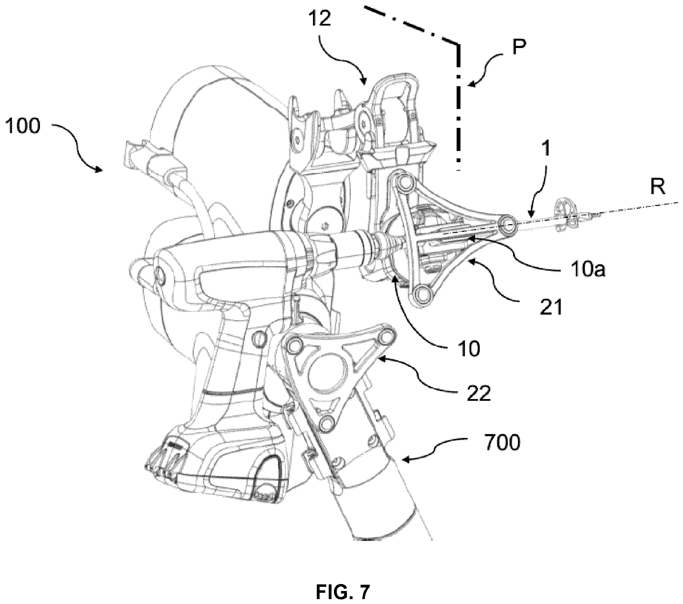

To that end, an end effector 10 comprising a surgical tool guide 10a (see FIG. 7) or holding a surgical tool 1 (see FIG. 2), which in some embodiments is intended to cut, drill, burr or ream an anatomical structure along at least one target axis is used by a user such as a surgeon.

In some embodiments, the cutting tool 1 may be replaced with an impacting tool (not shown). The impacting tool may be used to impact an implant component into the anatomical structure along the same, or a different, target axis.

In the following description and in the appended drawings, the surgical tool 1 is considered to be a rotary cutting tool having a rotation axis R which is the operative axis of the surgical tool. However, this is only for sake of illustration and without any intent to limit the disclosure with this embodiment.

The end effector 10 is held by a robotic device 100 which comprises an actuation unit 11 comprising preferably three or four motorized degrees of freedom. However, the actuation unit can include any number of degrees of freedom greater than three.

The end effector is coupled to the actuation unit by a planar articulation designated under reference 12 throughout the set of drawings, the planar articulation being configured to constrain the movement of the end effector within a single plane.

In some embodiments, as shown in FIG. 2, the rotation axis R of the rotary cutting tool may be contained in the plane P of the planar articulation 12, or in a plane parallel to said plane. In other embodiments, as shown in FIG. 7, the rotation axis R of the rotary cutting tool may be perpendicular to the plane P of the planar articulation 12.

In embodiments with only three or four motorized degrees of freedom there may not be sufficient degrees of freedom for the actuation unit to align the rotation axis of the cutting tool with the target axis.

However, as will be explained in more details below, if the actuation unit comprises three motorized degrees of freedom, the robotic device and planar articulation allow constraining the rotation axis of the rotary cutting tool inside a plane containing the target axis. If the actuation unit comprises four degrees of freedom, the rotation axis may be constrained to be parallel to the target axis. In both cases, a user manipulating the end effector is able, based on information computed by a control unit and provided to the user by a user interface in the form of visual, acoustic or haptic signals, to adjust the position and orientation of the rotation axis of the cutting tool with the target axis. In this manner, the user only has to manually move the cutting tool within a plane to align its rotation axis R with the desired trajectory. This operation is simpler than fully controlling the tool position manually, in six degrees of freedom.

The robotic device 100 is connected to a control unit 400 that controls the actuation unit.

Said control unit typically comprises power supply, AC/DC converters, motion controllers to power the motors of the actuation unit, fuses, real-time control system interface circuits.

The system also comprises a tracking unit 200, such that the relative pose of the device and the anatomical structure to be cut is tracked in real time and is shared between a real time control unit and a planning system.

At least one coordinate system 20 is attached to the anatomical structure while at least one coordinate system 21, 22 is attached to the end effector and/or to the robotic device. In some embodiments, a coordinate system 23 may be attached to the surgical tool.

The tracking unit measures the relative motions between the coordinate systems in real time. Real time means high frequencies greater than twenty Hertz, preferably in the range of one hundred to five hundred Hertz, with low latency, ideally less than five milliseconds.

The data obtained by the tracking unit are transferred to the control unit 400 via any suitable connection, with wires or wireless, with low latency.

The real-time control unit is able to carry out the proposed real-time control algorithms at a reasonably high frequency with low additional latency.

The real-time control unit computes in real time the position of the end effector with respect to a plane containing the target axis depending on said measured pose.

The control unit 400 and tracking unit 200 may be arranged in a cart 500 that can be moved in the operating room. They can be also mounted on separate carts, articulated holding arms, lighting systems, or the tracking unit can be also mounted directly on the anatomical structure or on some parts attached to the robotic device. For example, the end effector can rigidly support an electromagnetic emitter while electromagnetic sensors can be attached to the anatomical structure.

The system may also comprise a visual user interface 300 that is intended to display feedback information to a user. The feedback information may comprise indication about a deviation (distance and/or angle) between the rotation axis of the rotary cutting tool and the target axis, before the anatomical structure is cut.

Said user interface 300 may advantageously comprise a screen, which may be located on a cart in the operating room, e.g. on the same cart 500 as the control unit and tracking unit, or on a separate cart, or attached to the walls or the celling of the operating room.

In addition to or instead of said screen, the user interface may comprise an indicator that is arranged on the robotic device itself to provide information to the user. Said indicator can be made of LEDs arranged to indicate arrows, numbers or letters, or a miniature display.

Alternatively or in addition to the above mentioned embodiments, the user interface may be configured to generate acoustic or haptic signals configured to provide guidance to the user, for example via variable sound level or variable frequency depending on the deviation (distance and/or angle) between the rotation axis of the rotary cutting tool and the target axis.

A surgical system wherein the control unit, tracking unit and/or user interface are embedded in the robotic device itself may be an alternate embodiment provided that the embedded units are powered by a sufficiently powerful power supply or battery with size and weight that do not hinder the manipulation of the robotic device by the user. For example, micro cameras can be attached to the base of the actuation unit and markers can be attached to the anatomical structure and to the end effector.

### Surgical tool

In preferred embodiments, the surgical tool may be a rotary cutting tool.

The rotary cutting tool comprises a handpiece including at least one motor and an instrument configured to be rotated about a rotation axis (which is the operative axis of the tool) by the at least one motor when powered. The instrument may be, for example, a burr, a drill, a reamer, a screw, a K-wire, or a pin.

The handpiece may include at least one power switch connected to an energy source to provide power to the at least one motor or to stop powering the at least one motor. Said at least one power switch may be connected to the control unit so as to impede powering the motor(s) as long as the rotation axis is not aligned with the target axis.

In other embodiments, the surgical tool may be a manual tool, such as an impactor.

It should be noted that, in one surgical intervention, several surgical tools may be used in sequence. For example, in a first stage a rotary cutting tool may be used to create a cavity of a prosthesis about a target axis, and in a second stage the prosthesis is fitted into the cavity thanks to an impactor along the same or another target axis.

### Actuation unit

In preferred embodiments, the actuation unit comprises three or four motorized degrees of freedom, but the actuation unit may have other numbers of degrees of freedom.

The actuation unit may have a serial architecture, a parallel architecture or a mixed serial and parallel architecture.

According to an embodiment, the actuation unit 11 has a serial architecture made of a plurality of mobile segments, comprising from three to four motorized degrees of freedom, at least two of which being rotational degrees of freedom orthogonal to each other. In the present text, the term "axis" designates the geometric rotation or translation axis corresponding to said degree of freedom. Besides, the axes and segments are numbered with increasing numbers starting from the base (i.e. the part of the robotic device that remains stationary while the robotic device works) and towards the terminal part that is connected to the end effector; this type of numbering is conventional for serial robotic architectures.

In some embodiments, the actuation unit has three motorized rotational degrees of freedom.

In other embodiments, the actuation unit has two motorized rotational degrees of freedom and one or two motorized translational degrees of freedom.

According to another embodiment, the actuation unit presents a parallel architecture comprising a base and a platform selectively orientable relative to the base. To that end, the platform is linked to the base by a plurality of links each providing a degree of freedom in rotation, and if appropriate, also in translation. Various embodiments of parallel architecture exist, such as hexapods, Hayward wrists, Agrawal wrists, Gosselin's agile eyes, Tesar wrists, Cheng wrists, etc.

Depending on the architecture of the actuation unit, the components of the actuation unit may be integrated in an optimal way such that the robotic device remains as compact and light as possible while remaining strong enough to be able to hold the planar articulation and the end effector, as well as resisting to some normal pressure applied by the user when he/she manipulates the end effector.

As compared to such a surgical robot comprising at least six degrees of freedom, a compact actuation unit presents a lower inertia - especially according to the first axis - and thus a greater responsiveness required in particular to compensate for bone motion in real time.

### Holding arm

The robotic device may be held by a holding arm 700, especially if the robotic device is compact and lightweight, whatever the number of its degrees of freedom. The holding arm does not require any invasive action onto the patient while fully supporting the weight of the robotic device.

Such a holding arm supports the actuation unit and is suited to be connected to a mechanical support such as an operating table, a leg holder or mounted on a mobile cart which wheels can be blocked. A leg holder is an adjustable mechanism configured to maintain the leg in a given flexed position when the patient lies on the operating table.

The holding arm is made of several articulated segments using ball-and-socket joints, rotational and/or translational joints.

The holding arm is lockable, either manually by a knob (mechanical locking system) or actively by a dedicated actuator of a locking system. The locking system may be an electrical system, a piezoelectric system, a hydraulic system, a pneumatic system or a combination of such systems (e.g. a hydraulic cylinder driven by an electric motor). For example, company SMITH & NEPHEW sells a passive holding arm, actively lockable, named SPIDER^{™}. The actuator can be a button, a foot switch, a remote button, etc. To manipulate the robotic device, the user has to maintain the actuator activated until the desired pose of the robotic device has been achieved.

The holding arm supports the weight of the robotic device and maintains it in a rough positioning relative to the anatomical structure to be treated. It limits the movements of the user when operating the device - and, in advantageous embodiments, also damps movements of the user and/or the patient, vibrations of the end effector and reaction forces caused by movements of the actuation unit.

According to an embodiment, the holding arm is passive.

Advantageously, the holding arm may be braked progressively depending on the distance between the robotic device and a target position of the robotic device relative to a tracker fixed to the patient. For example, the braking force may be inversely proportional to the distance of the robotic device to its target position. Alternatively, one or several concentric volumes (e.g. cubes or spheres) may be defined around the target position of the robotic device. The braking force may adjust depending on the presence of the robotic device in one of said volumes. Thus, when the robotic device is close to the target position, the holding arm is braked and the user may receive a force-feedback information. Alternatively, feedback information may be provided in the form of a light, haptic or acoustic signal. For example, a variable flash frequency and/or intensity of a light signal may indicate the distance between the robotic device and its target position. Similarly, a variable frequency, repeat speed and/or amplitude of an acoustic signal may indicate such a distance. In any case, the braking is not full, so that the user is always able to manipulate the robotic device until its final desired position. The holding arm is then locked upon an action from the user (e.g. by operating the actuator, e.g. releasing or pushing a button). If the user wants to move the robotic device again, he/she has to operate the actuator again, which frees the holding arm - possibly with a braking force as described above. If a new target position of the robotic device is defined, new braking volumes are defined, and the braking is adjusted based on said new volumes.

In an embodiment, the holding arm is equipped with weights to counterbalance the weight of the control unit, as it is commonly used for carrying and placing microscopes in the surgical field for example.

In an embodiment, the holding arm has a vertical translation with a spring mechanism to compensate for the weight of the global system, then it has a serial architecture with a large planar structure made of three parallel and vertical axes. Each axis is equipped with a locking system.

Preferably, the connection between the holding arm and the actuation unit is as close as possible to the first segment of the actuation unit or to the center of gravity of the robotic device in order to minimize any lever-arm effect. The part of the actuation unit that is attached to the holding arm is called the base of the robotic device.

If the actuation unit has a serial architecture, the first segment of the actuation unit may be fixed relative to the holding arm. In such case, the second segment of the actuation unit is necessarily mobile relative to the first segment. This architecture is advantageous in that it minimizes the weight of the moving components of the actuation unit. As a result, the robotic device may be more responsive, which is favorable to real time control of the rotation axis of the tool. Alternatively, the first segment of the actuation unit may be mobile relative to the holding arm. In such case, the first and second segments are preferably embedded in a single housing.

If the actuation unit has a parallel architecture, the base of the actuation unit may be fixed relative to the holding arm.

As it will be explained in more details below, the actuation unit 11 is controlled by the control unit 400. The control unit may be integrated in the robotic device, or remote from the robotic device.

### Planar articulation

Several different architectures exist to implement a planar articulation. For example, the planar articulation can be made of only one rotation axis and then one translation axis that carries the end effector along its longitudinal direction. Otherwise, the planar articulation can be made of two or more rotation axes. Alternatively, the planar articulation can be made of two orthogonal translation axes and then a rotational axis. According to another embodiment, the planar articulation can be a slider in the form of an arch, including a rotation axis, and then a translation axis that carries the end effector.

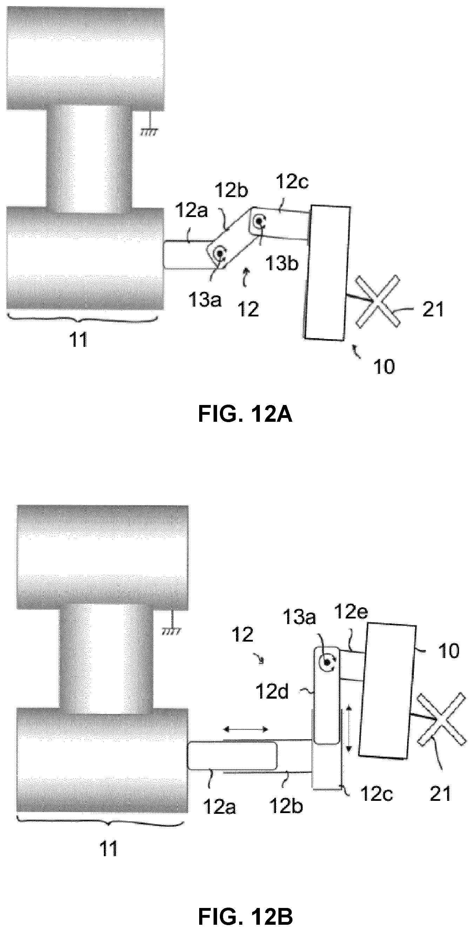

FIGS. 12A-12D illustrate various non-limitative embodiments of the planar articulation. Although the actuation unit is represented with a serial architecture and the end effector is represented as a saw, the invention could be implemented with any other type of actuation unit or end effector. The arrows indicate the degrees of freedom of the planar articulation in each embodiment.

FIG. 12A illustrates a planar articulation 12 comprising three linear segments 12a, 12b, 12c articulated about two rotation axes 13a, 13b that are orthogonal to the segments. The first segment 12a is rigidly coupled to the actuation unit 11 and the third segment is rigidly coupled to the end effector 10.

FIG. 12B illustrates a planar articulation 12 comprising five linear segments 12a-12e. The first and second segments 12a, 12b are in sliding engagement relative to each other, as well as the third and fourth segments 12c, 12d. The second and third segments 12b, 12c are rigidly coupled to each other and may form a single piece. The fourth and fifth segments 12d, 12e are articulated about a rotation axis 13a that is orthogonal to the segments. The first segment 12a is rigidly coupled to the actuation unit 11 and the fifth segment 12e is rigidly coupled to the end effector 10.

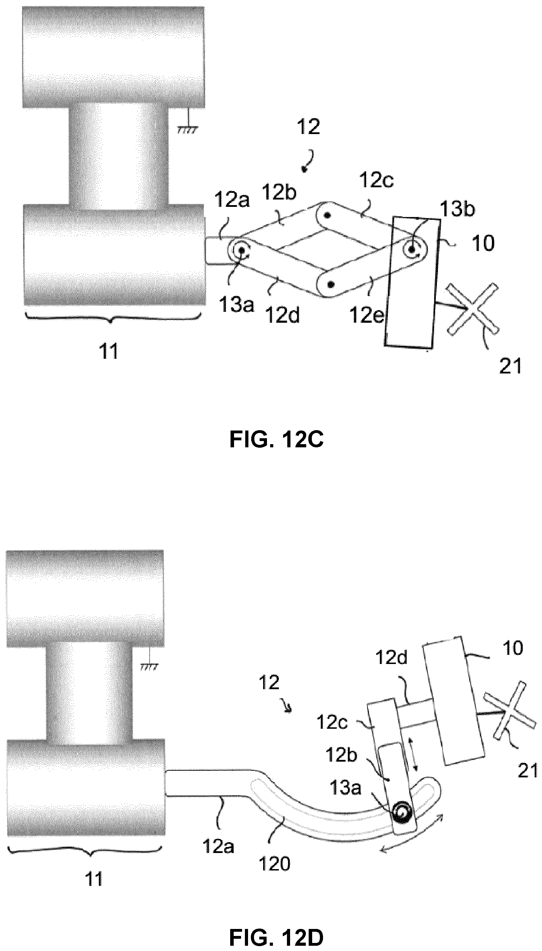

FIG. 12C illustrates a planar articulation 12 comprising five linear segments 12a-12e. The first segment 12a is rigidly coupled to the actuation unit 11. Two pairs of segments (respectively 12b-12c and 12d-12e) are connected to form a parallelogram. The segments 12b, 12d are articulated on the first segment 12a by a rotation axis 13a that is orthogonal to the segments. The segments 12c, 12e are coupled to the end effector 10 by a rotation axis 13b. The segments of each pair 12b-12c and 12d-12e are articulated about a respective axis that is orthogonal to said segments.

FIG. 12D illustrates a planar articulation 12 comprising a first segment 12a including a curved slot 120, a second linear segment 12b coupled to the first segment 12a via an axis 13a orthogonal to said segments. The axis 13a is able to slide within the curved slot 120, and the second segment 12b is rotatable relative to the axis 13a. A third linear segment 12c is in sliding engagement with the second segment, and rigidly connected to a fourth linear segment 12d that carries the end effector. The third and fourth linear segments 12c, 12d may form a single piece.

Advantageously, the end effector can be reversibly coupled to the planar articulation. Preferably, especially in the case where the end effector is not intended to receive a tracker, the attachment means for the end effector provides reproducible fixation.

In preferred embodiments, the planar articulation is passive, meaning that the articulation is not motorized and can be freely manipulated by the user. One advantage of such a passive articulation is to preserve all the perceptions of the user when the cutting tool or other device is manipulated in the bone. Thanks to the planar articulation, the user remains free to move the end effector within the plane of the planar articulation, thereby enjoying his/her usual feeling when accomplishing the surgical gesture. This also provides for a greater safety of use of the robotic device, since the user's intervention is always required to perform the cut. For example, surgeons are used to freely manipulate a drill or a reamer and detect when the instrument has reached a desired depth in the bone, and this perception is fully preserved with a passive planar articulation that has very low friction at its joints. In this respect, the planar articulation is advantageously provided with bearings that provide such a low friction for the various degrees of freedom.

The planar articulation may advantageously be made of lightweight and compact components so as to limit its bulkiness and thus to allow placing the end effector in any desired pose relative to the patient. As will be explained in more detail below, even if such a lightweight structure lacks stiffness, the invention provides for a control unit that is configured to implement a control loop that allows compensating for any offset between the real pose of the end effector and its theoretical one (i.e. the pose assuming that the planar articulation is perfectly stiff and always maintaining the end effector within the intended plane). The control loop uses a continuous tracking of the end effector.

In addition, the planar articulation may comprise a locking system for locking each of its degrees of freedom once a desired configuration has been achieved.

As an alternative to the locking system of the planar articulation, the robotic device may advantageously comprise a locking system for locking the end effector in a fixed position relative to the actuation unit when the robotic device has to move to a new position.

Various locking systems may be used (mechanical, magnetic, etc.).

One potential advantage of such a locking system of the planar articulation or of the end effector is that the end effector is maintained fixed relative to the terminal component of the actuation unit when the robotic device is being moved, which avoids any undesired movement of the end effector during this movement, which could cause the end effector to hit the patient, the user or an element of the operating room. In addition, with the locking system of the end effector, the actuation unit and end effector form a compact assembly, which is easier to move to a new desired position.

The locking unit may be provided with detectors allowing detecting whether the end effector is locked into the rest position or not.

The control unit may be configured to allow movement of the actuation unit only if such a locking into the rest position is detected.

It is to be noted that, since the end effector is equipped with a tracker, the control unit may allow movement of the actuation unit only if the tracking data show that the end effector is distant enough from the patient so as to avoid hitting him/her during said movement.

It is possible to make the actuation unit and planar articulation sterile components, to be sterilized before each intervention. But, in a preferred embodiment, the actuation unit with its cables and equipped with the planar articulation are covered by a single-use sterile drape. Additional components of the system can be also protected under the sterile drape. This has the advantage of facilitating and reducing cost of manufacturing and design, but also of being used easily for multiple consecutive surgeries without requiring re-sterilization of the device. The end effector itself is sterile, like any conventional surgical tool. Typically, it is sterilized before each intervention using autoclave. Different types of mechanical adaptors between the sterile drape and the end effector can be provided. Such adaptor does not require a very precise reproducible fixation if the saw contains a tracking element (described in more detail below), which increases the accuracy of the global system. The sterile drape covers the planar articulation to facilitate the design and manufacturing of the device. For example, this design allows the use of ball-bearings mechanisms that would be difficult to autoclave.

### Overview of the operation of the system

Before cutting the anatomical structure, the user plans the intervention on the planning system, based on pre-operative and/or intra-operative medical images and data.

This planning step allows determining at least one target axis suited to perform the cut of (or other operation on) the anatomical structure. Planning is typically specific to each application, hip, shoulder, etc.

In some embodiments, the planning step also allows determining a target point or area on the anatomical structure. For example, the target point may be located at the outer surface of the anatomical structure and located on the target axis; such a target point may thus be considered as an entry point for the rotary cutting tool. In other situations, the target area may be defined as the opening of a cavity of the anatomical structure (e.g. the acetabulum for the pelvis). The target point or area may not have to be determined very accurately by the control unit; if it is visible by the user, the user may visually position the tip of the rotary cutting tool to the target point or to the center of the target area.

In some embodiments, the planning step may also allow determining a cutting depth, corresponding to the position along the target axis at which the rotary cutting tool has to be stopped, the planned cutting being achieved. Otherwise, the user may also determine visually whether the cutting depth has been attained.

Said target axis and, if any, the entry point or area and the cutting depth, are known in the coordinate system of the anatomical structure. Since coordinate systems of the end effector and the anatomical structure are tracked in real time by the localization unit, the control unit can determine in real time the position and orientation of the rotation axis of the tool with respect to the target axis, and, if any, the entry point or area.

Besides, the control unit is configured to activate the actuation unit to maintain the planar articulation parallel to the target axis (if the actuation unit comprises four motorized degrees of freedom) or parallel to a plane containing said target axis (if the actuation unit comprises only three degrees of freedom), while compensating potential relative movements of the anatomical structure relative to the robotic device. Said relative movements may be caused by the user, by a surgical tool or by the patient.

In some embodiments, the planar articulation is configured to constrain movement of the rotation axis R of the rotary cutting tool inside a single plane P (see FIG. 2), which is the motion plane of the planar articulation or a plane parallel to said motion plane. The end effector may be coupled directly to the surgical tool or to a guide tube allowing guiding translation of the surgical tool that is held by the user.

In other embodiments, the planar articulation is configured to constrain movement of the rotation axis R of the rotary cutting tool perpendicular to a single plane P (see FIG. 7), which is the motion plane of the planar articulation. The end effector may be coupled directly to the surgical tool or to a guide tube 10a allowing guiding translation of the surgical tool that is held by the user. Such a guide tube may be especially necessary if the actuation unit does not comprise a degree of freedom allowing moving the surgical tool toward or away from the anatomical structure. In such case, the guide tube allows the user to manually translate the surgical tool toward or away from the anatomical structure.

Since there exist a plurality of planes containing the target axis, an arbitrary plane has to be selected among said plurality of planes. In some embodiments, said arbitrary plane may be selected as being relevant on an anatomical point of view; this may allow the user to better interpret the information displayed by the user interface and have a greater control of the execution of the intervention in view of the planning. For example, the user may thus remain free to adjust an angle of version based on his observation of the anatomical situation. In other embodiments, the arbitrary plane may be selected to be as horizontal as possible. The horizontality may be determined by a gravity sensor, which allows detecting a vertical direction. For example, in an optical localization unit, the stereoscopic camera may have such a sensor, which allows calculating the plane whose normal is the closest to the vertical direction and which contains the target trajectory. In practice, a simple vector product allows determining the orientation of this plane). The advantage of such a "most horizontal possible" plane is that it minimizes the user's efforts because the robotic device carries most of the weight of the rotary cutting tool. The arbitrary plane may change over time.

Based on planning and localization information received from the control unit, the user interface may display at least a representation of the target axis and a representation of the rotation axis of the cutting tool. In some embodiments, the user interface may also display a representation (e.g. more or less detailed model) and/or images of the anatomical structure. For example, said images may be CT slices viewed in multiple orientations to manage cutting (e.g. reaming/drilling/burring) depth properly. Angles between the rotation axis and the target axis can also be displayed by the user interface to better guide the user (especially if he wants to deviate from the planned position and orientation). Said CT slices may be adjusted automatically to follow the orientation of the tool: for example, the user interface may display two perpendicular slices (two views) containing the rotation axis of the rotary cutting tool.

In some embodiments, the user interface may display reaming/drilling/burring depth delta in a color scale mode (e.g. with a darker color assigned to the model of the anatomical structure as the depth increases).

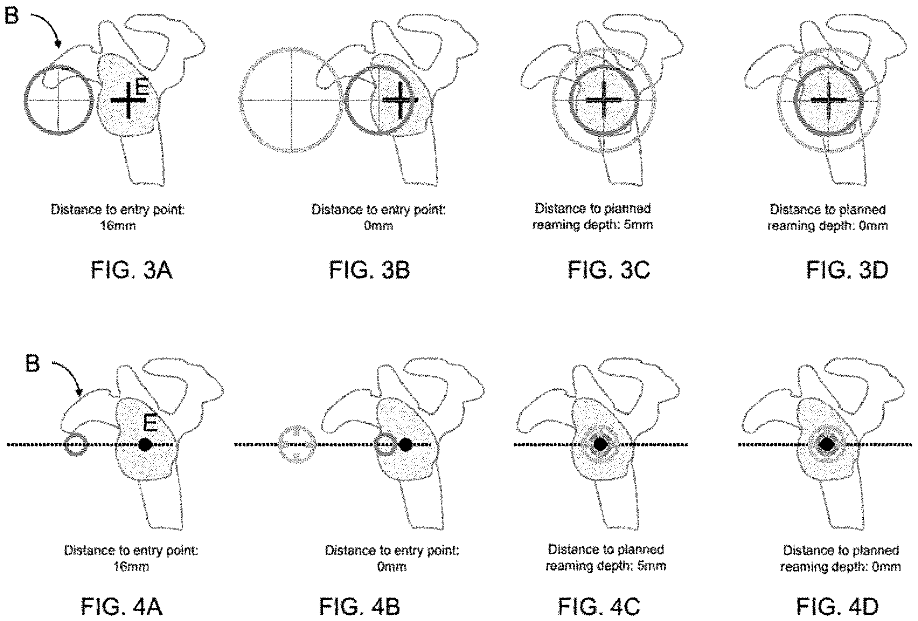

The information displayed by the user interface may take various forms, as shown non-limitatively in FIG. 3A-7B in the configuration wherein the rotation axis is constrained in a plane parallel to the motion plane of the planar articulation.

FIGS. 3A-3D illustrate the information displayed by the user interface in various steps of the positioning of the rotary cutting tool, in a first embodiment. The anatomical structure is represented schematically (although not shown, CT slices may be also used), with a target point E. As shown in FIG. 3A, in a first step, the tip of the cutting tool (represented by the center of the crosshair) is at a certain distance from the target point E, the target axis passing through point E and being perpendicular to the plane of the drawing sheet. As shown in FIG. 3B, in a second step, the tip of the cutting tool is at a null distance from the target point E. The crosshair already present in FIG. 3A still represents the tip of the cutting tool; the additional crosshair represents the opposite end of the cutting tool (e.g. the connection with the handpiece or the back of the handpiece). The line connecting the centers of the crosshairs thus represents the rotation axis of the cutting tool. In this position, the rotation axis is thus not aligned with the target axis. As shown in FIG. 3C, in a third step, the crosshairs are coaxial, which shows that the rotation axis is aligned with the target axis. At this stage, the user may switch on the rotary cutting tool to cause the tool to rotate and begin cutting the anatomical structure. As shown in FIG. 3D, in a fourth step, the planned cutting depth has been attained, which may be detected either by visual observation by the user or by navigating the tip of the cutting tool by the control unit. At this stage, the user may switch off the rotary cutting tool (or the control unit may automatically switch it off).

FIGS. 4A-4D illustrate the information displayed by the user interface in various steps of the positioning of the rotary cutting tool, in a second embodiment. The anatomical structure is represented schematically (although not shown, CT slices may be also used), with a target point E. As shown in FIG. 4A, in a first step, the tip of the cutting tool (represented by the center of the circle) is at a certain distance from the target point E, the target axis passing through point E and being perpendicular to the plane of the drawing sheet. As shown in FIG. 4B, in a second step, the tip of the cutting tool is at a null distance from the target point E. The circle already present in FIG. 4A still represents the tip of the cutting tool; the additional crosshair represents the opposite end of the cutting tool (e.g. the connection with the handpiece or the back of the handpiece). The line connecting the center of the crosshair and the center of the circle thus represents the rotation axis of the cutting tool. In this position, the rotation axis is thus not aligned with the target axis. As shown in FIG. 4C, in a third step, the crosshair and the circle are coaxial, which shows that the rotation axis is aligned with the target axis. At this stage, the user may switch on the rotary cutting tool to cause the tool to rotate and begin cutting the anatomical structure. As shown in FIG. 4D, in a fourth step, the planned cutting depth has been attained, which may be detected either by visual observation by the user or by navigating the tip of the cutting tool by the control unit. At this stage, the user may switch off the rotary cutting tool (or the control unit may automatically switch it off).

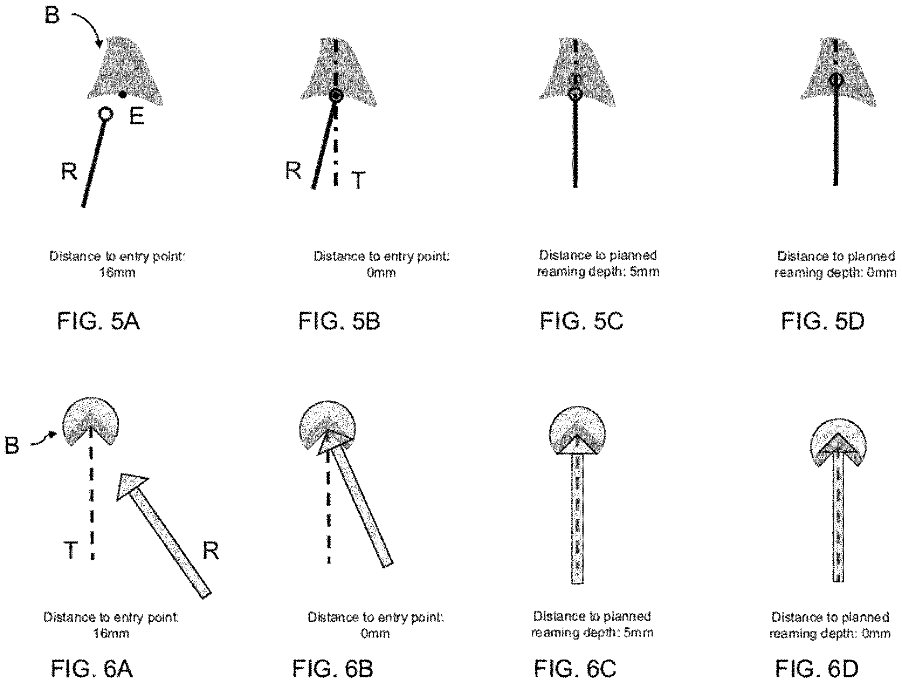

FIGS. 5A-5D illustrate the information displayed by the user interface in various steps of the positioning of the rotary cutting tool, in a third embodiment. The anatomical structure is represented schematically (although not shown, CT slices may be also used), with a target point E. As shown in FIG. 5A, in a first step, the tip of the cutting tool (represented by the center of the circle) is at a certain distance (e.g. 16 mm) from the target point E. As shown in FIG. 5B, in a second step, the tip of the cutting tool is at a null distance from the target point E. The rotation axis (represented by the plain line extending from the circle) is not aligned with the target axis (represented by the dotted line passing through point E). If the surgical tool has a pointed tip, the user may slightly indent the surface of the anatomical structure with the pointed tip in order to fix the tip to the anatomical structure, and then rotate the surgical tool about the tip until aligning the rotation axis with the target axis. As shown in FIG. 5C, in a third step, the plain and dotted lines coincide, which shows that the rotation axis is aligned with the target axis. The distance to the planned reaming depth (e.g. 5 mm) may be displayed. At this stage, the user may switch on the rotary cutting tool to cause the tool to rotate and begin cutting the anatomical structure toward an end point identified by the second circle. As shown in FIG. 5D, in a fourth step, the planned cutting depth has been attained (the distance to the planned reaming depth being null), the circle representing the tip of the cutting tool coinciding with said second circle. At this stage, the user may switch off the rotary cutting tool (or the control unit may automatically switch it off).

FIGS. 6A-6D illustrate the information displayed by the user interface in various steps of the positioning of the rotary cutting tool, in a fourth embodiment. The anatomical structure is represented schematically (although not shown, CT slices may be also used), a target point E being defined at the bottom of a cavity, along the target axis which is represented by the dotted line. As shown in FIG. 6A, in a first step, the tip of the cutting tool (represented by the tip of the arrow) is at a certain distance (e.g. 16 mm) from the target point. As shown in FIG. 6B, in a second step, the tip of the cutting tool is at a null distance from the target point E. The rotation axis (represented by the plain line extending from the circle) is not aligned with the target axis. If the surgical tool has a pointed tip, the user may slightly indent the surface of the anatomical structure with the pointed tip in order to fix the tip to the anatomical structure, and then rotate the surgical tool about the tip until aligning the rotation axis with the target axis. As shown in FIG. 6C, in a third step, the plain and dotted lines coincide, which shows that the rotation axis is aligned with the target axis. At this stage, the user may switch on the rotary cutting tool to cause the tool to rotate and begin cutting the anatomical structure in a zone represented by a different color. As shown in FIG. 6D, in a fourth step, the planned cutting depth has been attained (the distance to the planned reaming depth being null), the tip of the arrow representing the tip of the cutting tool reaching the limit of the cut zone. At this stage, the user may switch off the rotary cutting tool (or the control unit may automatically switch it off).

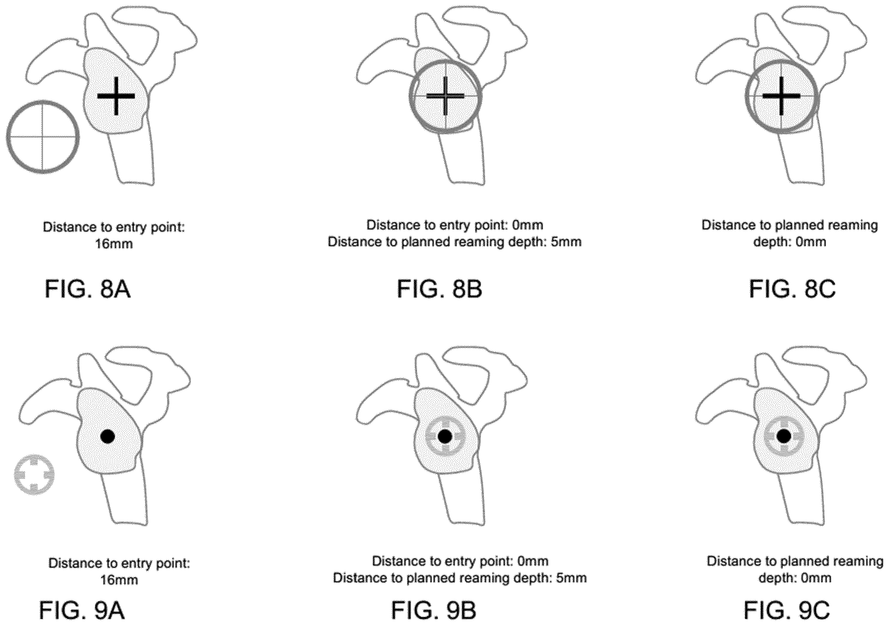

In the configuration wherein the rotation axis is constrained in a plane perpendicular to the motion plane of the planar articulation, it is easier for the user to align the rotation axis with the target axis. The information displayed by the user interface may take various forms, as shown non-limitatively in FIG. 8A-9C.

FIGS. 8A-8C illustrate the information displayed by the user interface in various steps of the positioning of the rotary cutting tool, in a first embodiment. The anatomical structure is represented schematically (although not shown, CT slices may be also used), with a target point E. As shown in FIG. 8A, in a first step, the tip of the cutting tool (represented by the center of the crosshair) is at a certain distance from (e.g. 16 mm) the target point E, the target axis passing through point E. Both the rotation axis and the target axis are perpendicular to the plane of the drawing sheet. As shown in FIG. 8B, in a second step, the tip of the cutting tool is at a null distance from the target point E, the center of the crosshair coinciding with the target point E. The rotation axis and the target axis, that are still both perpendicular to the plane of the drawing sheet, are thus aligned. The distance to the planned reaming depth (e.g. 5 mm) may be displayed. At this stage, the user may switch on the rotary cutting tool to cause the tool to rotate and begin cutting the anatomical structure. As shown in FIG. 8C, in a third step, the planned cutting depth has been attained (the distance to the planned reaming depth being null). At this stage, the user may switch off the rotary cutting tool (or the control unit may automatically switch it off).

FIGS. 9A-9C illustrate the information displayed by the user interface in various steps of the positioning of the rotary cutting tool, in a second embodiment. This second embodiment differs from the first one only on the way the target point is represented (by a small disk instead of a cross) and the way the rotation axis is represented (by a circle with two pairs of diametrically opposite segments instead of a crosshead). The guidance provided by the user interface is similar to the one described with reference to FIGS. 8A-8C.

The user has to maintain the operative axis aligned with the target axis until the planned operation has been achieved, the actuation unit constraining the operative axis to remain in a plane containing the target axis. In case the control unit detects a deviation of the operative axis from the target axis, it may cause the user interface to generate a warning signal, or reduce the power of the cutting tool.

The achievement of the planned cutting depth may be detected by navigating the tip of the surgical tool with the control unit. An indication of the current cutting depth may thus be provided by the user interface. Alternatively, the user may be able to detect that the planned cutting depth has been attained by visual observation of the surgical scene.

As mentioned above, although the illustrated embodiments of the user interface relate to information displayed on a screen, the user interface may not comprise a screen but may consist in any other signal emitting means. For example, the user interface my comprise one or more LEDs arranged on the robotic device and adapted to present a variable color and/or blinking frequency depending on the distance of the operative axis relative to the target axis and/or the target point, if any. Alternatively, the user interface may comprise sound emitting means adapted to generate sounds with variable level and/or frequency depending on the distance between the operative axis and the target axis, or haptic feedback means.

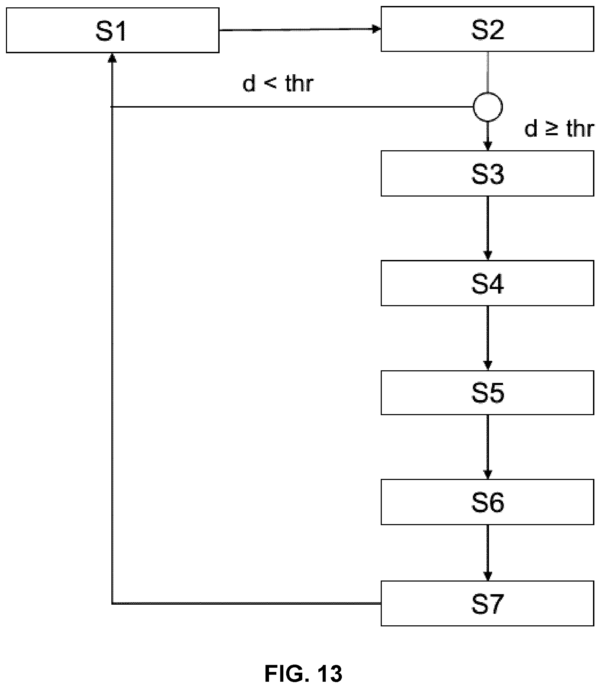

FIG. 13 is a flowchart describing the control loop allowing the motion compensation.

In step S1, new poses of the robotic device, the end effector and the anatomical structure are determined using localization information provided by the trackers.

In step S2, a deviation d between a plane comprising the rotation axis of the tool and parallel to the motion plane of the planar articulation and an arbitrary plane comprising the target axis is computed.

If the deviation d is less than a threshold thr, the end effector can be operated and a new pose of the robotic device and anatomical structure is determined (step S1).

If the deviation d is greater than or equal to the threshold thr, then in step S3 the plane comprising the rotation axis and the arbitrary plane comprising the target axis are projected in the coordinate system of the robotic device.

In step S4, a correction matrix Terr corresponding to a rigid transformation between the motion plane of the planar articulation and the plane comprising the rotation axis of the tool is computed.

In step S5, the arbitrary plane comprising the target axis is updated with Terr.

In step S6, a new attitude of the robotic device is computed to reach the arbitrary plane comprising the target axis. This computation determines the movements to be applied by the motors of the actuation unit.

In step S7, the motors of the actuation unit are activated in accordance with step S'6.

Then, the new position of the robotic device and anatomical structure is determined (step S1).

From this base algorithm, further improvements have proven to enhance the behavior of the robotic device:
- spatially filtering the positions of the various elements (for instance thanks to a Kalman filter or equivalent);
- averaging the estimation of Terr in a given time frame, for instance thanks to quaternion averaging techniques. This allows reducing the potential oscillations due to small inconsistencies between the transformation estimation and the more complex reality of the mechanical links.

The correction matrix Terr may vary depending on the current extension of the planar articulation and therefore it is not constant. It also depends on the mechanical backlash and flexion of the planar articulation, the position of the robotic device, and other factors. The correction matrix is calculated in real time, such that the deviation of Terr between two calculations is not significant, considering reasonable motions of the tool by the user. This method of correction is extremely precise and efficient for compensating any mechanical defects, backlash and errors in the model.

It is to be noted that this compensation loop also allows at least partially compensating for backlash and flexion of the actuation unit.

### Example 1: Hip surgery

Hip surgery may include the positioning of a cup implant in the acetabulum. To that end, the acetabulum has to be reamed by a reamer to suitable shape and dimensions to create a volume adapted to receive the cup. The reamer has a hemispherical shape substantially corresponding to the cup size and comprises a plurality of cutting teeth arranged on its outer surface, such that rotation of the reamer causes bony material from the acetabulum to be removed.

Thanks to the substantially hemispherical shapes of the acetabular cavity and the reamer, the reamer may be able to position itself in the acetabular cavity. Thus, a surgeon only has to control the orientation of the reamer and the reaming depth.

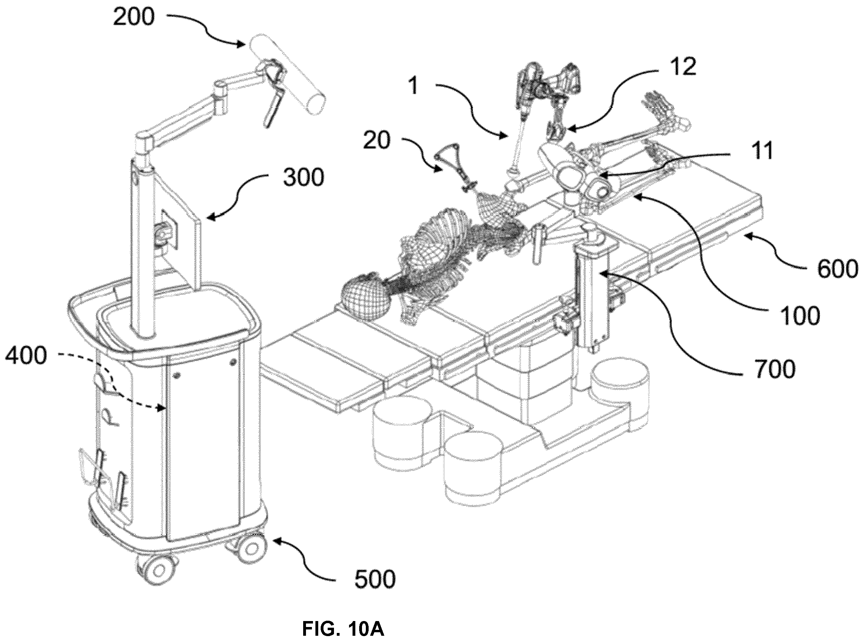

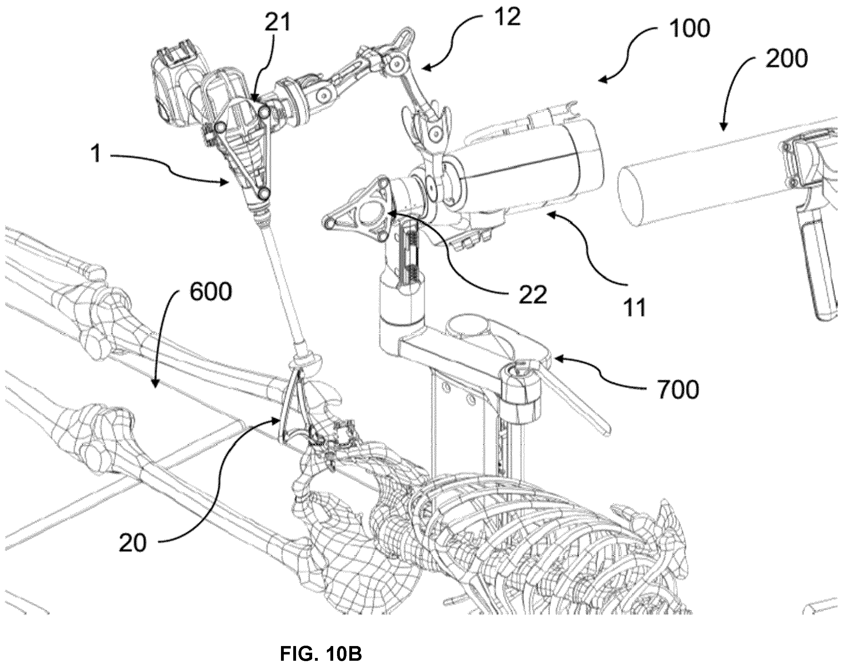

During the intervention, the patient may be in supine position or in lateral decubitus position on an operating table 600, as shown in FIGS. 10A-10B.

The robotic device 100 is positioned next to the patient. In some embodiments, the robotic device is fixed at an end of a lockable holding arm 700 having its opposite end attached to the operating table or to another fixed element in the operating room.

The reamer 1 may be attached to the planar articulation 12; alternatively, a guide tube may be attached to the planar articulation and the reamer inserted into the guide tube. Trackers 20, 21 are attached at least to the patient's acetabulum and to the reamer (or the guide tube, if any).

The reamer trajectory may be planned at any time before the intervention or during the intervention before actuating the robotic device. Said trajectory is defined by a target axis, which corresponds to the axis of the cup to be placed in the acetabular cavity. The trajectory may further be defined by the position of a target point, which is the final position of the reamer tip.

Said planned trajectory may be generated by the control unit or generated by another system and then transferred to the control unit by any suitable communication means.

The localization device is capable of determining in real time the position and orientation of the rotation axis of the reamer and of the acetabular cavity.

Based on said planned trajectory and on localization data from the localization device, the control unit is capable of determining a position and orientation of the rotation axis relative to a plane containing the target axis.

When the user is ready to begin the reaming, he switches on the robotic device so as to cause the actuation unit to orient the reamer in such a way as the rotation axis of the reamer is parallel to an arbitrary plane containing the target axis.

Since the robotic device is held by the holding arm, the user only has to manipulate the reamer which is freely movable inside a plane thanks to the planar articulation. However, along said movement generated by the user, the actuation unit constrains the rotation axis to remain parallel to the arbitrary plane containing the target axis. In addition, if the patient moves, the actuation unit moves the planar articulation to maintain the rotation axis parallel to the plane containing the target axis.

To bring the rotation axis of the reamer in alignment with the target axis, the user may use the user interface that displays the position and orientation of the rotation axis relative to the target axis. The user may thus directly see on the user interface the effect of the movement imparted to the reamer.

When the user can see that the rotation axis of the reamer is aligned with the target axis and the reamer is located in the opening of the acetabular cavity, the user may switch on the reamer to remove bony material from the acetabulum. In some embodiments, said switching on may be impeded by the control unit if the rotation axis of the reamer is not aligned with the target axis.

Once the planned cutting depth has been attained, the user may switch off the reamer. Otherwise, the reamer can be switched off automatically by the control unit, if the reamer is wired or has a wireless connection means to the control unit, such as Bluetooth/Wifi, etc.).

The acetabular cup may then be placed into the reamed cavity.

The surgical robotic system may advantageously also be used to guide an impactor to impact the acetabular cup. To that end, the user may remove the reamer from the guide tube and insert the impactor into the guide tube. The position and orientation of the guide tube may be aligned with a target axis in a similar way as above. Said target axis may be identical to the target axis planned for the reaming step, or different.

### Example 2: Shoulder surgery

Shoulder surgery may include the positioning of a glenoid implant in the glenoid. To that end, the glenoid has to be reamed by a reamer to suitable shape and dimensions to create a volume adapted to receive the glenoid implant. The reamer has a hemispherical shape substantially corresponding to the glenoid implant size and comprises a plurality of cutting teeth arranged on its outer surface, such that rotation of the reamer causes bony material from the acetabulum to be removed.

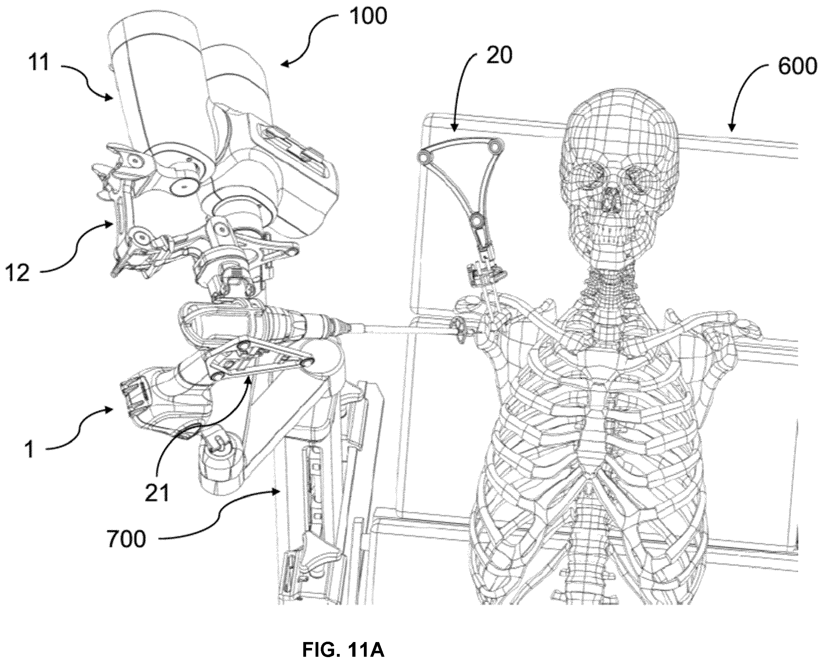

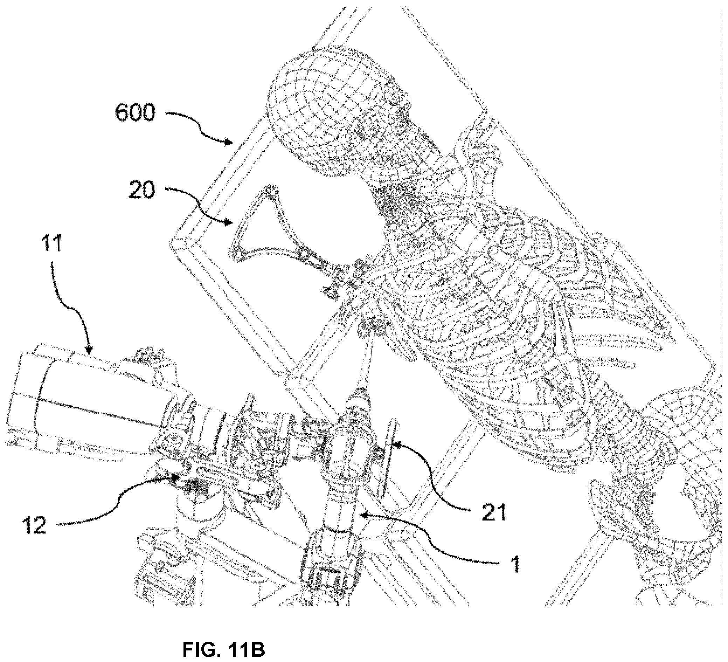

During the intervention, the patient may be in beach chair position on an operating table 600, as shown in FIGS. 11A-11B.

The robotic device 100 is positioned next to the patient. In some embodiments, the robotic device is fixed at an end of a lockable holding arm 700 having its opposite end attached to the operating table or to another fixed element in the operating room.

The reamer 1 may be attached to the planar articulation 12; alternatively, a guide tube may be attached to the planar articulation and the reamer inserted into the guide tube. Trackers 20, 21 are attached at least to the patient's acetabulum and to the reamer (or the guide tube, if any).

The reamer trajectory may be planned at any time before the intervention or during the intervention before actuating the robotic device. Said trajectory is defined by a target axis and an entry point, which is the point on the surface of the glenoid through which the reamer has to enter the glenoid.

Said planned trajectory may be generated by the control unit or generated by another system and then transferred to the control unit by any suitable communication means.

The localization device is capable of determining in real time the position and orientation of the rotation axis of the reamer relative to the target axis and the target point.

Based on said planned trajectory and on localization data from the localization device, the control unit is capable of determining a position and orientation of the rotation axis relative to a plane containing the target axis.

When the user is ready to begin the reaming, he switches on the robotic device so as to cause the actuation unit to orient the reamer in such a way as the rotation axis of the reamer is parallel to an arbitrary plane containing the target axis.

Since the robotic device is held by the holding arm, the user only has to manipulate the reamer which is freely movable inside a plane thanks to the planar articulation. However, along said movement generated by the user, the actuation unit constrains the rotation axis to remain parallel to the arbitrary plane containing the target axis. In addition, if the patient moves, the actuation unit moves the planar articulation to maintain the rotation axis parallel to the plane containing the target axis.

To bring the rotation axis of the reamer in alignment with the target axis, the user may use the user interface that displays the position and orientation of the rotation axis relative to the target axis and to the target point. The user may thus directly see on the user interface the effect of the movement imparted to the reamer. As mentioned above, when the tip of the reamer is in contact with the target point, he may slightly indent the surface of the glenoid with the tip and use it as a fixed pivot point to align the rotation axis with the target axis.

When the user can see that the rotation axis of the reamer is aligned with the target axis and the reamer tip is on the target point, the user may switch on the reamer to remove bony material from the acetabulum. In some embodiments, said switching on may be impeded by the control unit if the rotation axis of the reamer is not aligned with the target axis.

Once the planned cutting depth has been attained, the user may switch off the reamer. Otherwise, the reamer can be switched off automatically by the control unit, if the reamer is wired or has a wireless connection means to the control unit, such as Bluetooth/Wifi, etc.).

The disclosure is not limited to the examples provided above, but may also be applied in all interventions in orthopedics requiring guiding a trajectory of a surgical tool, including for example the following applications: nail and screw positioning in trauma surgery, screw placement in spine surgery.

## Claims

1. System for guiding a surgical tool (1) comprising an operative axis with respect to an anatomical structure according to a planned trajectory defined by a target axis (T), comprising:
- a robotic device (100) comprising:
∘ an end effector (10) comprising the surgical tool or configured to be coupled to the surgical tool;
∘ an actuation unit (11) comprising at least three motorized degrees of freedom;
∘ a planar articulation (12) coupling the end effector (10) to the actuation unit (11), the planar articulation being configured to constrain movement of the operative axis (R) of the surgical tool inside a single plane;
- a localization device (200) configured to determine in real time a position and orientation of the operative axis (R) with respect to a coordinate system of the anatomical structure;
- a user interface (300);
- a control unit (400) coupled to the localization device (200), the actuation unit (11) and the user interface (300);
wherein the control unit (400) is configured to:
- based on the planned trajectory and on localization data from the localization device, determine a position and orientation of the operative axis (R) relative to a plane containing the target axis (T);
- control the actuation unit to constrain the operative axis (R) inside the plane containing the target axis (T) while a user moves the end effector (10) closer to the target axis (T);
- generate by the user interface (300) at least one signal related to the position and orientation of the operative axis (R) relative to the target axis (T).

2. System for guiding a surgical tool (1) comprising an operative axis (R) with respect to an anatomical structure according to a planned trajectory defined by a target axis (T), comprising:
- a robotic device (100) comprising:
∘ an end effector (10) comprising the surgical tool or configured to be coupled to the surgical tool;
∘ an actuation unit (11) comprising at least three motorized degrees of freedom;
∘ a planar articulation (12) coupling the end effector to the actuation unit, the planar articulation being configured to constrain movement of the operative axis of the rotary cutting tool to be perpendicular to a single plane;
- a localization device (200) configured to determine in real time a position and orientation of the operative axis with respect to a coordinate system of the anatomical structure;
- a user interface (300);
- a control unit (400) coupled to the localization device, the actuation unit and the user interface;
wherein the control unit is configured to:
- based on the planned trajectory and on localization data from the localization device, determine an orientation of the planar articulation relative to the target axis;
- control the actuation unit to constrain the plane of the planar articulation to be perpendicular to the target axis while a user moves the end effector closer to the target axis;
- generate by the user interface at least one signal related to the position of the operative axis relative to the target axis.

3. System according to claim 1 or claim 2, wherein the actuation unit (11) comprise at least four motorized degrees of freedom and the control unit is configured to control the actuation unit to constrain the operative axis to be parallel to the target axis while the user moves the end effector closer to the target axis.

4. System according to any one of claims 1 to 3, wherein the planned trajectory is further defined by a target point or area (E), and wherein the control unit is configured to generate by the user interface a signal related to the position of the operative axis (R) of the surgical tool relative to the target point or area.

5. System according to claim 4, wherein based on localization data from the localization device, the control unit is configured to determine a position of a distal tip of the surgical tool relative to the target point or area.

6. System according to claim 5, wherein the user interface is configured to generate a signal related to the position of the distal tip of the surgical tool relative to the target point or area.

7. System according to any one of claims 4 to 6, wherein the surgical tool is a powered tool, the target point defines an end of the trajectory of the surgical tool and the control unit is configured to switch off the surgical tool when the distal tip of the surgical tool reaches the target point.

8. System according to any one of claims 1 to 7, wherein the system comprises a first tracker (21, 23) configured to be rigidly attached to the end effector or to the surgical tool and a second tracker (20) configured to be rigidly attached to the anatomical structure.

9. System according to any one of claims 1 to 8, wherein the control unit is configured to determine that the operative axis is aligned with the target axis.

10. System according to claim 9, wherein the control unit is configured to determine that the operative axis is aligned with the target axis when an angle between the operative axis of the cutting tool and the target axis is less than 1°.

11. System according to any one of claims 1 to 10, further comprising a lockable holding arm (700) supporting the actuation unit (11).

12. System according to any one of claims 1 to 11, wherein the surgical tool is a powered tool and the control unit is configured to impede switching on the rotary cutting tool as long as the operative axis is not aligned with the target axis.

13. System according to any one of claims 1 to 12, wherein the surgical tool comprises a drill, a reamer, a burr, a screw, a K-wire, or a pin.

14. System according to any one of claims 1 to 13, wherein the surgical tool is an impactor.

15. Surgical system according to the combination of claims 13 and 14, wherein the end effector (10) comprises a guide tube (10a) adapted to alternately guide the drill, reamer or burr and the impactor.
